# EUROPÄISCHE PATENTSCHRIFT

(11) **EP 1 928 810 B1**
(45) Veröffentlichungstag und Bekanntmachung des Hinweises auf die Patenterteilung: **21.12.2011**
(21) Anmeldenummer: 06791883.9
(22) Anmeldetag: 07.09.2006
(51) Int. Cl.: C07C 17/02, C07C 19/045

(54) **VERFAHREN ZUM BETRIEB EINER DESTILLATIONSKOLONNE ZUR REINIGUNG VON 1 ,2-DICHLORETHAN UND ZUR GEKOPPELTEN NATRONLAUGEEINDAMPFUNG**
METHOD OF OPERATING A DISTILLATION COLUMN FOR PURIFYING 1,2-DICHLOROETHANE AND FOR COUPLED SODIUM HYDROXIDE SOLUTION EVAPORATIVE CONCENTRATION
PROCEDE PERMETTANT DE FAIRE FONCTIONNER UNE COLONNE DE DISTILLATION POUR L'EPURATION DE 1, 2-DICHLORETHANE ET POUR L'EVAPORATION CONCOMITANTE D'UNE LESSIVE DE SOUDE

(30) Priorität: 15.09.2005 DE 102005044177
(43) Veröffentlichungstag der Anmeldung: 11.06.2008
(73) Patentinhaber: ThyssenKrupp Uhde GmbH, 44141 Dortmund (DE); Vinnolit GmbH & Co. KG, 84504 Burgkirchen (DE)
(72) Erfinder: PETERSEN, Sven, 65779 Kelkheim (DE); BENJE, Michael, 64289 Darmstadt (DE); KAMMERHOFER, Peter, 84508 Burgkirchen (DE)
(86) Internationale Anmeldenummer: PCT/EP2006/008703
(87) Internationale Veröffentlichungsnummer: WO 2007/031223

(56) Entgegenhaltungen:
- WO-A2-01/34542
- DE-A1- 4 039 960

## Beschreibung

Die Erfindung richtet sich auf ein Verfahren zur Herstellung von 1,2-Dichlorethan, im folgenden als EDC bezeichnet, welches überwiegend als Zwischenprodukt der Herstellung von monomerem Vinylchlorid, im folgenden als VCM bezeichnet, dient, woraus letztlich Polyvinylchlorid, PVC, hergestellt wird. Bei der Umsetzung von EDC zu VCM entsteht Chlorwasserstoff HCl. EDC wird daher bevorzugt aus Ethen C₂H₄ und Chlor Cl₂ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

Cl₂ + C₂H₄ → C₂H₄Cl₂ (Rein-EDC) + 218 kJ/Mol (1)

C₂H₄Cl₂ (Spalt-EDC) → C₂H₃Cl (VCM) + HCl - 71 kJ/Mol (2)

C₂H₄ + 2 HCl + ½ O₂ → C₂H₄Cl₂ (Roh-EDC) + H₂O + 238 kJ/Mol (3)

Das Verfahren zur Herstellung von VCM mit ausgewogener HCl-Bilanz, im folgenden kurz "ausgewogenes VCM-Verfahren" genannt, besitzt:
- eine Direktchlorierung, in der aus Ethen C₂H₄ und Chlor Cl₂ der eine Teil des benötigten EDC erzeugt wird und als sogenanntes Rein-EDC abgegeben wird; die Verwertung der bei dieser Direktchlorierung erzeugten Reaktionswärme ist zentraler Bestandteil der Erfindung;
- eine Oxichlorierung, in der aus Ethen C₂H₄, Chlorwasserstoff HCl und Sauerstoff O₂ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen;
- eine EDC-Pyrolyse, in der das Rein-EDC mit dem Feed-EDC zusammengeführt und in der das dann Spalt-EDC genannte Gemisch thermisch gespalten wird; das erhaltene Spaltgas enthält VCM, Chlorwasserstoff HCl und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Das in der Direktchlorierung benötigte Chlor Cl₂ wird üblicherweise in einer Anlage zur Elektrolyse aus Natriumchlorid NaCl erzeugt. Als Koppelprodukt entsteht hierbei Natronlauge NaOH mit einer Konzentration von ca. 33 %. Wegen der hohen Giftigkeit des erzeugten Chlors Cl₂ ist man bestrebt, einen weiten Transport möglichst zu vermeiden. Oft wird daher das für die Direktchlorierung benötigte Chlor Cl₂ in unmittelbarer Nähe einer Anlage zur Direktchlorierung erzeugt.

Die in der EDC-Pyrolyse im Spaltgas entstehenden Begleitstoffe setzen bekanntlich die Produkt-Reinheit des VCM herab. Die Reinigung des VCM durch Entfernung der Begleitstoffe ist dementsprechend aufwendig. Daher wird ein weitestgehend von Verunreinigungen befreites Spalt-EDC in der EDC-Pyrolyse eingesetzt. Aus der großen Zahl von Techniken, wie die entsprechenden und nachteiligen Nebenprodukte und/oder Begleitstoffe vermieden bzw. gegebenenfalls abgereinigt werden können, sei vor allem auf die Schrift WO 01/34542 A2 verwiesen, insbesondere auf den dort gewürdigten Stand der Technik. Hierbei konnte gezeigt werden, dass die Reaktionswärme, die beim Direktchlorierungsverfahren durch Umsetzung von Ethen C₂H₄ und Chlor Cl₂ in flüssigem EDC frei wird, ausreicht, um die Reinigungskolonnen für erzeugtes EDC im ausgewogenen VCM-Verfahren zu betreiben.

Nachteilig ist bei dem dort vorgestellten Verfahren jedoch, dass die zur Beheizung der Reinigungskolonnen eingesetzte Reaktionswärme die Abführung einer korrespondierenden Wärmemenge zur Kondensation der Brüden erfordert. Diese Abführung geschieht nach dem herkömmlichen Stand der Technik üblicherweise mittels Kühlwasser, welches in großer Menge bereitgestellt werden muss. Aber auch dann, wenn die Reinigungskolonnen nicht mit Abwärme aus der Reaktionswärme der Direktchlorierung betrieben werden, muss viel Kühlwasser zur Kondensation der Brüden der Reinigungskolonnen bereitgestellt werden.

Die Aufgabe der Erfindung ist es daher, die Abwärmenutzung des ausgewogenen VCM-Verfahrens weiter zu optimieren und den Bedarf an Kühlwasser deutlich zu verringern.

Die Erfindung löst die Aufgabe dadurch, dass die Kondensationswärme der Brüden, die bei der destillativen Abtrennung von leichter als EDC siedenden Komponenten anfallen, zumindest teilweise für die Eindampfung der bei der Chlorherstellung als Koppelprodukt erzeugten Natronlauge genutzt wird. Eine solche Destillationskolonne ist als sogenannte Leichtsiederkolonne üblicherweise Bestandteil des ausgewogenen VCM-Verfahrens. Im ausgewogenen VCM-Verfahren sind diese abzutrennenden Komponenten zum überwiegenden Teil Reaktionswasser, welches in der Oxychlorierung in die Leichtsiederkolonne eingetragen wird und dann von EDC zu trennen ist, sowie organische, leichter als EDC siedende Bestandteile.

Insbesondere in entlegenen Gebieten spielen die Transportkosten für den Abtransport der bei der NaCl-Elektrolyse erzeugten Natronlauge NaOH eine wichtige Rolle. Diese Transportkosten können deutlich gesenkt werden, wenn die bei einer Konzentration von ca. 33 % erzeugte Lauge auf 50 % eingedampft wird. Eine derartige Anlage zur Eindampfung von Natronlauge NaOH kann z.B. unter Vakuum bei einem Absolutdruck von 133 mbar und einer Temperatur von 60 °C betrieben werden. Auch für den Fall, dass EDC-Herstellung und NaCl-Elektrolyse nicht nebeneinander angeordnet sind, ist es lohnend, die erzeugte, 33-%ige Natronlauge zunächst zur EDC-Herstellungsanlage zu transportieren, und dort mittels einer EDC-betriebenen Vakuumeindampfungsanlage eine NaOH-Eindampfung vorzunehmen. Die Eindampfung kann natürlich auch auf andere Konzentrationen als auf 50 % erfolgen, je nach Abnehmerwunsch und Abwärmeanfall.

In einer Ausgestaltung der Erfindung wird zumindest ein Teil des aus Chlor und Ethylen in einer Direktchlorierung gebildeten EDCs zur Beheizung der Destillationskolonne zur Entfernung von Wasser und leichter als EDC siedenden Komponenten eingesetzt.

In einer weiteren Ausgestaltung der Erfindung kann zumindest ein Teil desjenigen EDCs, welches zur Beheizung der Destillationskolonne zur Entfernung von Wasser und leichter als EDC siedenden Komponenten eingesetzt wurde, anschließend noch als Wärmeträger für die Eindampfung von Natronlauge verwendet werden. Dies ist möglich, weil das Temperaturniveau der Beheizung der Leichtsiederkolonne höher ist, als das der Natronlaugeeindampfung, und daher noch eine weitere Temperaturabsenkung der in der Beheizung der Destillationskolonne kondensierten EDC-Brüden unter Energieabgabe in der Natronlaugeeindampfung möglich ist.

Die zum Einsatz kommenden apparativen Einrichtungen zur Übertragung der Kondensationsenergie bestehen aus denen des bekannten, ausgewogenen VCM-Verfahrens und den Einrichtungen für die einzudampfende Natronlauge NaOH. Für letzteres kommt hauptsächlich ein stehender Rohrbündelwärmetauscher mit 2 festen Rohrplatten und einem NaOH-Sumpfteil zum Einsatz, bei dem die Natronlauge NaOH rohrinnenseitig von oben nach unten und Leichtsieder-Brüden auf der Außenseite der Rohre geführt wird. Die Wärmeübertragung im Rohrbündel findet im Gleichstrom statt. Der oben auf das Rohrbündel aufgegebene Brüden kondensiert dabei und kann unten flüssig abgezogen werden.

Die Wärmeübertragung kann auch zweckmäßig sowohl mittels eines eingesteckten Wärmetauscherbündels im Natronlaugesumpf erfolgen, als auch mittels eines außerhalb des Natronlaugesumpfes gelegenen und im Umlauf betriebenen Wärmetauschers, z.B. vom Kettle-Typ, erfolgen.

Alle oben beschriebenen Methoden sind auch additiv bzw. in Kombination anwendbar. Soll das Verfahren in Kombination mit anderen Verfahren, die ebenfalls Natronlaugeeindampfung vorsehen, angewendet werden, und dabei verschiedene Brüden gleichzeitig genutzt werden, kann das Rohrbündel horizontal geteilt werden. Selbstverständlich ist darauf zu achten, dass die einzelnen Brüdenströme verschiedener Destillationskolonnen nicht miteinander vermischt werden dürfen.

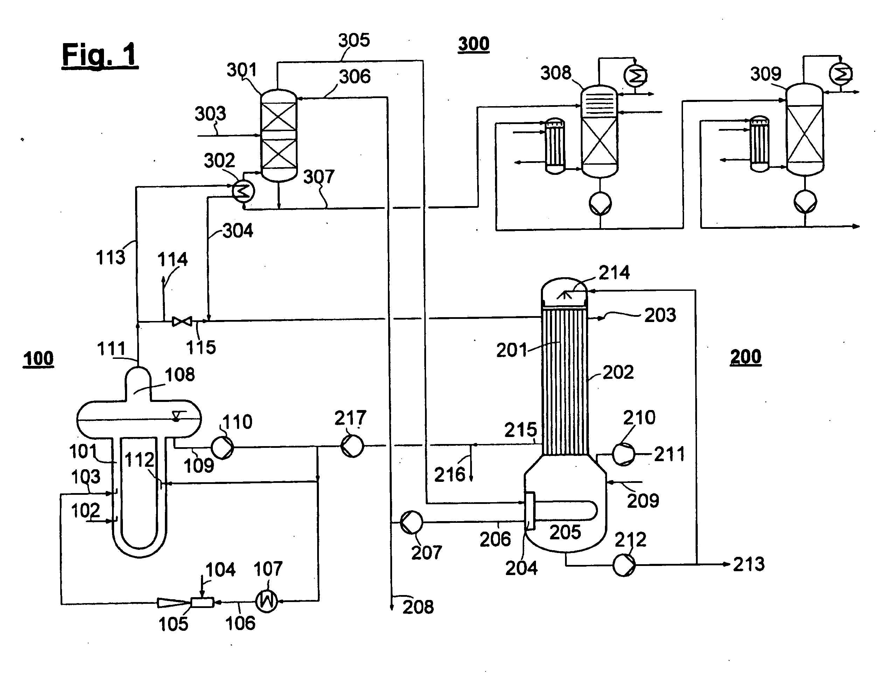

Fig. 1 zeigt hierbei in Anlehnung an die Lehre der WO 01/34542 A2 anhand eines vereinfachten Verfahrensfließbildes eine Verschaltung einer EDC-Reinigung 300, die in einer Anlage nach dem ausgewogenen VCM-Verfahren arbeitet, mit einer Direktchlorierung 100 und einer Natronlaugeeindampfung 200, wobei
- die Brüden der Leichtsiederkolonne 301 der EDC-Reinigung 300, dem Hauptanspruch entsprechend, in einer Natronlaugeeindampfung 200 kondensiert werden,
- die Beheizung der Leichtsiederkolonne 301, Anspruch 2 entsprechend, durch EDC-Brüden der Direktchlorierung 100 erfolgt, und
- das Kondensat der EDC-Brüden, Anspruch 3 entsprechend, nach der Beheizung der Leichtsiederkolonne 301 ebenfalls zur Natronlaugeeindampfung 200 verwendet wird.

Die Direktchlorierung 100 besteht aus einer flüssigkeitsgefüllten Schlaufe 101, einer Einspeisung von Ethylen 102, einer Zugabe von in EDC gelöstem Chlor 103, wobei das Chlorgas 104 vorher im Injektor 105 in flüssigem EDC 106 gelöst wurde, welches im EDC-Kühler 107 zuvor zur Verbesserung der Löslichkeit auf eine niedrige Temperatur abgekühlt wurde, ferner einem Ausgasgefäß 108, einer Abzugsvorrichtung für flüssiges EDC 109 mit einer EDC-Umwälzpumpe 110, einer Abzugsvorrichtung für dampfförmiges EDC 111 und einer Zuführstelle von Umlauf-EDC 112, wobei die jeweiligen Zuführstellen und Abzugsvorrichtungen aus praktischen Gründen auch mehrfach ausgeführt sein können. In der flüssigkeitsgefüllten Schlaufe 101 reagieren Chlor und Ethylen miteinander zu siedendem EDC, welches im Ausgasgefäß 108 zusammen mit nicht reagierten Ausgangsstoffen und inertem Begleitgas ausdampft.

Das dampfförmige EDC 113 wird zum Teil dem Sumpfaufkocher 302 der Leichtsiederkolonne 301 zugeführt. Der Anteil des zuzuführenden dampfförmigen EDC 113 am gesamten aus der Direktchlorierung abgezogenen EDC richtet sich dabei nach der Betriebsweise der Leichtsiederkolonne 301, vor allem nach der jeweiligen Wasserfracht im Roh-EDC 303 und den Reinheitsanforderungen für die Trennung, und beträgt im Normalfall ca. ein Fünftel bis ein Drittel. Das übrige dampfförmige EDC 114 wird üblicherweise an anderer Stelle im ausgewogenen VCM-Verfahren eingesetzt oder kann ebenfalls zur Natronlaugeeindampfung genutzt werden.

Typischerweise beträgt die Sumpftemperatur der Leichtsiederkolonne 301 etwa 115 °C, während das dampfförmige EDC bei etwa 120 bis 125 °C aus der Direktchlorierung 100 abgezogen werden kann. Wegen dieser geringen Temperaturdifferenz kann auf diese Weise über die reine Kondensation hinaus dem EDC kaum noch Abwärme zum Betrieb der Leichtsiederkolonne 301 entnommen werden, weshalb das EDC-Kondensat 304, welches noch dampfförmiges EDC und inerte Gasbestandteile enthält, mit einer Temperatur von ca. 120°C als Mehrphasengemisch aus dem Sumpfaufkocher 302 abgeführt wird.

Das EDC-Kondensat 304 wird, gegebenenfalls nach Vermischung mit dampfförmigem EDC 115, in die Natronlaugeeindampfung 200 gegeben, welche als stehendes Rohrbündel mit erweitertem Sumpfteil ausgeführt ist. Dort wird es in den Mantelraum 201 des Rohrbündelwärmetauschers 202 gegeben, während im Innenraum der Rohre eine Fallfilmverdampfung der Natronlauge bei ca. 60 °C stattfindet. Nicht kondensierbare Bestandteile werden über den Inertgasabzug 203 abgeführt. Hierbei ist durch geeignete technische Maßnahmen sicher zu stellen, dass sich im Mantelraum 201 des Rohrbündelwärmetauschers 202 kein explosionsfähiges Gasgemisch bilden kann. Solche Maßnahmen sind dem Fachmann bekannt und nicht Gegenstand der Erfindung.

Der Leichtsieder-Brüden 305 der Leichtsiederkolonne 301 wird ebenfalls zur Natronlaugeeindampfung 200 geführt, wo er im Einsteckkühler 204, der im Sumpfteil 205 der Natronlaugeeindampfung 200 angebracht ist, kondensiert wird. Das erhaltene Leichtsieder-Kondensat 206 wird von der Kondensatpumpe 207 zum Einen als Leichtsieder-Rückfluss 306 zum Kopf der Leichtsiederkolonne 301 zurückgeführt und zum Anderen als Abwasser 208 abgeleitet. Aufgrund der hohen Temperaturdifferenz zwischen der Natronlaugeeindampfung 200 mit ca. 60 °C und der Kopftemperatur der Leichtsiederkolonne 301, die bei ca. 90°C liegt, ist eine kompakte Bauform möglich.

Die 33-%ige Natronlauge 209 wird in den Sumpfteil 205 der Natronlaugeeindampfung 200 eingegeben und unter Vakuum eingedampft. Die Druckhaltung erfolgt durch die Vakuumpumpe 210, die den freiwerdenden Wasserdampf 211 abführt. Die Natronlaugepumpe 212 führt einen Teil der auf ca. 50 % konzentrierten Natronlauge als Produkt-NaOH 213 ab und fördert einen anderen Teil zum Natronlauge-Verteiler 214, der die einzudickende Natronlauge in das Rohrinnere des Rohrbündelwärmetauschers 202 aufteilt.

Das EDC-haltige Sumpfprodukt 307 der Leichtsiederkolonne 301 wird in die Hochsiederkolonne 308 geleitet und dort sowie in der nachfolgenden Vakuumkolonne 309 in bekannter Weise weiter aufgereinigt. Auch für die Beheizung dieser Kolonnen ist Reaktionswärme der EDC-Herstellung vorteilhaft einsetzbar, was aber nicht Gegenstand der vorliegenden Erfindung ist.

Dem aus dem Mantelraum 201 des Rohrbündelwärmetauschers 202 abgezogenen Rein-EDC 215 wird Produkt-EDC 216 abgezweigt, das übrige Rein-EDC wird mit der EDC-Pumpe 217 dem Kreislauf der Direktchlorierung 100 zugeleitet und mit dem flüssigen EDC 109 vereinigt.

Hierbei ist die Führung der Brüdenströme aus der Direktchlorierung 100 und Leichtsiederkolonne 301 nur beispielhaft dargestellt. Ebenso wäre es möglich, einen externen Aufkocher in der Natronlaugeeindampfung 200 einzusetzen oder den Mantelraum 201 zu teilen. Hierbei besteht Freiheit, in welchen Teil welcher Brüden zur Wärmenutzung eingebracht werden soll, was der Fachmann entsprechend der örtlichen Gegebenheiten jeweils wirtschaftlich zu optimieren hat, wobei er natürlich darauf zu achten hat, die verschiedenen Qualitäten an EDC-Strömen nicht miteinander zu vermischen.

Zur Veranschaulichung dient das folgende Zahlenbeispiel auf der Basis einer Simulationsrechnung: Zugrunde gelegt wird dabei eine Anlage mit einer Kapazität von 400 000 Jahrestonnen EDC. Bei einer Anlage dieser Größe lassen sich in der Leichtsiederkolonne bei einem Kopfdruck von 1,15 bar absolut und einer Temperatur von ca. 91 °C ca. 3,9 MW thermische Leistung für die Natronlaugeeindampfung gewinnen, womit sich ca 6,8 t/h Natronlauge (als 100 % gerechnet) von 33 auf 50 Gewichtsprozent aufkonzentrieren lassen. Für den Betrieb der Leichtsiederkolonne werden im Sumpfaufkocher 5,2 MW aus der Direktchlorierung eingebracht, was 30°% der gesamten Reaktionsenergie ausmacht.

### Liste der verwendeten Bezugszeichen

- **100**: **Direktchlorierung**
- 101: flüssigkeitsgefüllte Schlaufe
- 102: Einspeisung von Ethylen
- 103: gelöstes Chlor
- 104: Chlorgas
- 105: Injektor
- 106: flüssiges EDC
- 107: EDC-Kühler
- 108: Ausgasgefäß
- 109: flüssiges EDC
- 110: EDC-Umwälzpumpe
- 111: dampfförmiges EDC
- 112: Umlauf-EDC
- 113: dampfförmiges EDC
- 114: dampfförmiges EDC
- 115: dampfförmiges EDC
- **200**: **Natronlaugeeindampfung**
- 201: Mantelraum
- 202: Rohrbündelwärmetauscher
- 203: Inertgasabzug
- 204: Einsteckkühler
- 205: Sumpfteil
- 206: Leichtsieder-Kondensat
- 207: Kondensatpumpe
- 208: Abwasser
- 209: 33-%ige Natronlauge
- 210: Vakuumpumpe
- 211: Wasserdampf
- 212: Natronlaugepumpe
- 213: Produkt-NaOH
- 214: Natronlauge-Verteiler
- 215: Rein-EDC
- 216: Produkt-EDC
- 217: EDC-Pumpe
- **300**: **EDC-Reinigung**
- 301: Leichtsiederkolonne
- 302: Sumpfaufkocher
- 303: Roh-EDC
- 304: EDC-Kondensat
- 305: Leichtsieder-Brüden
- 306: Leichtsieder-Rückfluss
- 307: Sumpfprodunkt
- 308: Hochsiederkolonne
- 309: Vakuumkolonne

## Patentansprüche

1. Verfahren zum Betrieb einer Destillationskolonne zur Entfernung von Wasser und leichter als 1,2-Dichlorethan siedenden Komponenten aus 1,2-Dichlorethan, **dadurch gekennzeichnet, dass** zumindest ein Teil der Kondensationswärme der wasserhaltigen Brüden der Destillationskolonne für die Eindampfung von Natronlauge verwendet wird.

2. Verfahren nach Anspruch 1, **dadurch gekennzeichnet, dass** zumindest ein Teil des aus Chlor und Ethylen in einer Direktchlorierung gebildeten 1,2-Dichlorethans zur Beheizung der Destillationskolonne zur Entfernung von Wasser und leichter als 1,2-Dichlorethan siedenden Komponenten aus 1,2-Dichlorethan eingesetzt wird.

3. Verfahren nach Anspruch 2, **dadurch gekennzeichnet, dass** zumindest ein Teil des 1,2-Dichlorethans, welches zur Beheizung der Destillationskolonne zur Entfernung von Wasser und leichter als 1,2-Dichlorethan siedenden Komponenten eingesetzt wurde, anschließend als Wärmeträger für die Eindampfung von Natronlauge verwendet wird.

## Claims

1. Process for operating a distillation column for the removal of water and components which boil at a lower temperature than 1,2-dichloroethane from 1,2-dichloroethane, **characterised in that** at least part of the heat of condensation from the aqueous vapours from the distillation column are used for evaporation of the caustic soda.

2. Process according to claim 1, **characterised in that** at least part of the 1,2-dichloroethane formed from chlorine and ethylene in a direct chlorination unit is used to heat the distillation column for the removal of water and components which boil at a lower temperature than 1,2-dichloroethane from 1,2-dichloroethane.

3. Process according to claim 2, **characterised in that** at least part of the 1,2-dichloroethane used to heat the distillation column for the removal of water and components which boil at a lower temperature than 1,2-dichloroethane is subsequently used as a heat transfer fluid for the evaporation of caustic soda.

## Revendications

1. Procédé d'exploitation d'une colonne de distillation pour éliminer du 1,2-dichloroéthane l'eau et les composants présentant un point d'ébullition inférieur au 1,2-dichloroéthane, **caractérisé en ce qu'**au moins une partie de la chaleur de condensation des buées aqueuses est utilisée pour l'évaporation de la solution d'hydroxyde de sodium.

2. Procédé selon la revendication 1, **caractérisé en ce qu'**au moins une partie du 1,2-dichloroéthane produit par chloration directe d'éthylène avec du chlore est utilisée pour chauffer la colonne de distillation pour éliminer du 1,2-dichloroéthane l'eau et les composants présentant un point d'ébullition inférieur au 1,2-dichloroéthane.

3. Procédé selon la revendication 2, **caractérisé en ce qu'**au moins une partie du 1,2-dichloroéthane utilisé pour chauffer la colonne de distillation pour éliminer l'eau et les composants présentant un point d'ébullition inférieur au 1,2-dichloroéthane, est alors utilisé comme thermofluide pour l'évaporation de la solution d'hydroxyde de sodium.
